# EUROPEAN PATENT SPECIFICATION

(11) **EP 1 626 698 B1**
(45) Date of publication and mention of the grant of the patent: **13.08.2008**
(21) Application number: 04734270.4
(22) Date of filing: 21.05.2004
(51) Int. Cl.: A61K 6/08, A61C 5/12

(54) **A MATRIX BAND**
EIN MATRIZENBAND
MATRICE

(30) Priority: 23.05.2003 FI 20030779
(43) Date of publication of application: 22.02.2006
(73) Proprietor: Stick Tech OY, 20521 Turku (FI)
(72) Inventor: VALLITTU, Pekka, FI-21620 Kuusisto (FI); LASSILA, Lippo, FI-21630 Lielax (FI); YLI-URPO, Antti, FI-20660 Littoinen (FI); TEZVERGIL, Arzu, FI-20810 Turku (FI)
(74) Representative: Maskula, Silla Marjatta
(86) International application number: PCT/FI2004/000308
(87) International publication number: WO 2004/103318

(56) References cited:
- EP-A- 0 296 539
- EP-A- 0 459 361
- WO-A-02/100355
- US-A1- 2003 068 598
- US-B1- 6 186 790
- US-B1- 6 197 410

## Description

### FIELD OF THE INVENTION

This invention relates to a dental matrix band. The invention further relates to a dental restoration kit, a prepreg as well as to the use of the present matrix band.

### BACKGROUND OF THE INVENTION

The publications and other materials used herein to illuminate the background of the invention, and in particular, the cases to provide additional details respecting the practice, are incorporated by reference.

It is common dental practice to use metal or polymer matrix band in fabrication of dental restorations, such as fillings of composite resins. The matrix band gives support for the non-polymerized restorative composite resin before it is polymerized. Another meaning of the matrix band is to help the dentist to form the filling to the desired form, i.e. giving for instance good approximal contacts to the adjacent teeth. The dentist has used various types of matrix bands for many decades in the tooth filling process. Matrix bands have been described in the patent literature e.g. in US 1,255,109, US 2,310,448, US 2,594,367 and US 5,380,198. The matrix band is also referred to as matrix strip in the literature.

Document US 2003/0068598 discloses a dental device including a shapable prepreg that contains fibres, and at least one solid body attached to the prepreg, for use in the construction of a finished dental device. Document EP 296 539 discloses a matrix for use in the composite restoration of a posterior tooth proximal surface, the matrix being made of the same material as the composite restoration and intended to stay on place after the restoration is finished. Document US 6,186,790 presents prefabricated components for dental appliances that are preferably fabricated of a fiber-reinforced composite material comprising fibers impregnated with a polymer matrix.

The problem related to the state-of-the-art matrix bands relates to the removal of the matrix bands after the composite resin has been polymerized. The removal causes a gap between the filling and adjacent tooth. For example, typical thickness for a dental matrix band made of Mylar® (a polyester) film is 0,05 mm.

The matrix band should also be transparent in order facilitate the photopolymerization of the restorative composite resin through the matrix band. Metallic matrix bands do not facilitate photopolymerization through the matrix band.

One additional problem related to the dental fillings is recurrent cusp fractures besides the filling although the composite resin should support the cusps and reinforce the tooth by good adhesion of the composite resin restoration to the tooth.

### OBJECTS AND SUMMARY OF THE INVENTION

The object of this invention is to provide a matrix band that solves the above-mentioned problems. It is especially an object of the present invention to provide a matrix band that allows to minimize or to completely avoid the formation of a gap between the filling formed and the adjacent tooth.

A further object of this invention is to provide a matrix band that minimizes the cusp fractures besides the filling formed.

The above-mentioned problems are solved by the matrix band according to the present invention. A typical matrix band according to the invention is characterized in that it comprises fibers and a matrix, at least a portion of said matrix being at least partially uncured.

The present invention also relates to a dental restoration kit comprising a matrix band according to the present invention, a restorative dental composite and an adhesive.

The invention further relates to a prepreg for use as a matrix band as well as to the use of the present matrix band for the manufacturing of a dental restoration, a dental bridge or a dental crown and in dental and medical applications.

A typical prepreg according to the invention comprises fibers and a matrix, at least a portion of said matrix being at least partially uncured.

### DETAILED DESCRIPTION OF THE INVENTION

The invention is disclosed in the appended independent claims. The dependent claims define various embodiments of the invention.

A typical matrix band according to the present invention is characterized in that it comprises fibers and a matrix, at least a portion of said matrix being at least partially uncured.

In this application, by curing it is meant polymerization and/or crosslinking. By matrix, it is understood the continuous phase of the composition and by uncured matrix it is meant a matrix that is in its deformable state but that can be cured, i.e. hardened, to a non-deformable state. The uncured matrix may already comprise some long chains but it is essentially not yet polymerized and/or crosslinked. By partially uncured it is meant that the material is partly cured but still contains short chains that can be polymerized and/or crosslinked, and that the material is still in a deformable state. By prepreg, it is meant a semi-manufactured product, that is, a product that is no or only partly polymerized yet still deformable. The polymerization of a prepreg leads to a composite. The words "composite" and "cured prepreg" may be used interchangeably. "Dental restoration" is used as meaning typical dental fillings but also as a general term to include all dental repairs, such as crowns and bridges.

The matrix band according to the present invention thus comprises both fibers and a matrix. By the matrix, it is meant the resin material between and optionally on the fibers that wets the fibers. Some examples of suitable fibers and materials for the matrix are given below. At least a portion of said matrix is at least partially uncured, i.e. at least a portion of it can be further polymerized and/or crosslinked. Furthermore, said at least a portion of said matrix is capable of chemical and/or mechanical bonding, i.e. it can be polymerized and/or crosslinked, as said above, but it can also form mechanical bonding, such as interlocking or a structure known as interpenetrating networks. It may also be porous and thus capable of mechanical bonding.

Fiber-reinforced composite technology has been introduced to dentistry during late 1990's mainly for reinforcing dentures against cracks and to make fiber-reinforced composite fixed partial dentures. Its use in the present application, i.e. a matrix band, has however not been suggested earlier.

This invention thus consists of a combination of fiber-reinforced composite technologies to form a dental matrix band. With the present invention it is possible to provide a new approach to make tooth filling and at same time to reinforce the tooth structure to prevent the cusp fractures, or to stabilize tooth with existing minor fractures. Further advantages can also be achieved e.g. the prevention of marginal leakage between the filling and the tooth, when the fiber-reinforced composite is covering the border area between the filling and tooth.

The intention of the matrix band according to the present invention is to be attached to the filling composite resin and to form an integral part of the tooth-filling system after the filling composite resin has been cured. Thus, the matrix band is not removed after making the filling. The invention further benefits the tooth-filling system by offering protection for cusps against fractures by encapsulating the cusps with the matrix band and to minimize the microleakage by covering the border region of the filling and tooth. The matrix band can also be used to encapsulate old restorations and dental bridges. It is also especially suitable to be used to encapsulate frameworks of fiber-reinforced composite bridges to the abutment teeth.

The matrix band according to the present invention is preferably used on an untreated tooth, i.e. on tooth that has not been drilled. The surface of the tooth may have been previously etched or a priming may have been applied. These treatments are common in the art of dentistry and well known to persons skilled in the art.

The present invention thus solves the problems mentioned above, i.e. it provides a matrix band that allows to minimize or to completely avoid the formation of a gap between the filling formed and the adjacent tooth. Furthermore, it provides a matrix band that minimizes the cusp fractures besides the filling formed. The matrix band according to the present invention also provides for more surface of attachment for the dental restoration.

The matrix band according to the present invention is made of fiber-reinforced prepreg materials, for example such as those described in patent US 6,197,410 (herein incorporated by reference). According to an embodiment of the invention, the matrix band is made of woven glass fibers wetted with a highly viscous resin. It also possible that all of the resin matrix of the fiber-reinforced composite matrix band is at least partially in its non-cured form when the matrix band is placed on tooth. According to an embodiment, it is also possible to have the matrix of the matrix band to be in a cured form at one part of the band and no or partly cured form in another part of the band. In this latter case, it is preferred to have a cured matrix part, i.e. rigid part at margin of the band to help placing the matrix band into the gingival pocket. In the case of a cured matrix, it is preferred to have a porous polymer matrix allowing formation of interpenetrating polymer network bonding between the restorative composite resin and the matrix band to ensure attachment of the matrix to the composite filling. A polymer matrix of this type has been described in patent US 6,197,410. If the matrix of the matrix band is in its non-cured form when the matrix is used, then once the restorative composite resin (by restorative composite resin, it is meant the filling material of the tooth cavity) is cured, the attachment of the matrix band is based on the polymerization and/or crosslinking by free radical polymerization of the monomers of the matrix of the matrix band and on the polymerization and/or crosslinking of the monomers of the restorative composite resin, at the same time.

When the matrix band according to the present invention is used, it is placed totally or partially around the remaining tooth being in contact with the possible adjacent tooth. When the restorative resin is applied to the cavity, it becomes into contact with the matrix band. During the curing of the restorative composite resin, the matrix of the matrix band is also cured and attached to the composite. At the same time, the matrix band covers the tooth substance underneath and encapsulates the tooth. The encapsulation by the fiber-reinforced matrix band reinforces the tooth-filling system against fractures of tooth. The filling is preferably finished and polished in conventional ways and it is possible to cover the matrix band with restorative composite resin to increase the wear resistance of the surface of the matrix band.

When the matrix band according to the present invention is used as a part of a fiber-reinforced composite bridge, the matrix band is placed over the framework and abutment tooth to encapsulate the tooth, possible fillings of the tooth and partially or totally the framework of the bridge. The matrix band encapsulation can be made for the bridges and restorations made by direct technique, by indirect technique or by their combination. The matrix band will remain as an integral part of the tooth-bridge or more generally, tooth-restoration system.

It is also possible to overlay the matrix band that has been cured with a layer of restorative composite resin to reshape the tooth morphology to fulfill the needs of the occlusion. By this, the attached matrix band supports the composite resin that has been used to reshape the tooth.

The fiber or fibers used in the composition may be any fiber known *per se* that are compatible with the matrix used and a person skilled in the art will be able to readily assess which fiber is the most suitable for the intended application.

The fibers may for example be selected from a group consisting of inert glass fibers (such as S or E glass), bioactive glass fibers, silica fibers, quartz fibers, ceramic fibers, carbon/graphite fibers, aramid fibers, ceramic fibers, poly(p-phenylene-2,6-benzobisoxazole) fibers (PBO), poly(2,6-diimidazo(4,5-b4',5'-e)pyridinylene-1,4(2,5-dihydro)phenylene fibers (PIPD), polyolefin fibers, fibers prepared from copolymers of olefins, polyester fibers, polyamide fibers, polyacrylic fibers, sol-gel processed silica fibers, collagen fibers, cellulose fibers and modified cellulose fibers. Any combination of said fibers may be used. Poly(p-phenylene-2,6-benzobisoxazole) fibers and poly(2,6-diimidazo(4,5-b4',5'-e)pyridinylene-1,4(2,5-dihydro)phenylene fibers belong to a group called rigid-rod polymer fibers. It is obvious to a person skilled in the art that any other known fibers may be used in the present invention, provided it is possible to obtain a suitable adhesion between said fibers and matrix, in order to achieve the desired mechanical properties. In dental applications the most suitable fibers are, at the moment of filing this application, glass fibers due to their good cosmetic and esthetic properties and because the glass fibers allow light polymerization to be performed through the matrix band.

The fibers of the matrix band may be in any desired form, such as continuous fibers, chopped fibers or in the form of woven or nonwoven mat or sheet. The orientation of the fibers may be unidirectional, bidirectional, tridirectional or have a random orientation.

The matrix of the matrix band may be made of any suitable monomer or polymer or a mixture of them.

The matrix of the matrix band may comprise monomers selected from the group consisting of methyl acrylate, ethyl acrylate, propyl acrylate, isopropyl acrylate, n-hexyl acrylate, styryl acrylate, allyl acrylate, methyl methacrylate, ethyl methacrylate, propyl methacrylate, isopropyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diurethane dimethacrylate, acetoacetoxy ethyl methacrylate (AAEM), methacrylate functionalized dendrimers, other methacrylated hyperbranched oligomers, hydroxymethyl methacrylate, hydroxymethyl acrylate, hydroxyethyl methacrylate, hydroxyethyl acrylate, hydroxypropyl methacrylate, hydroxypropyl acrylate, tetrahydrofurfuryl methacrylate, tetrahydrofurfuryl acrylate, glycidyl methacrylate, glycidyl acrylate, triethylene glycol diacrylate, tetraethylene glycol dimethacrylate, tetraethylene glycol diacrylate, trimethylolethane trimethacrylate, trimethylolpropane trimethacrylate, pentaerythritol trimethacrylate, trimethylolethane triacrylate, trimethylolpropane triacrylate, pentaerythritol triacrylate, pentaerythritol tetramethacrylate, pentaerythritol tetra-acrylate, ethylene dimethacrylate, ethylene diacrylate, ethylene glycol dimethacrylate, diethylene glycol dimethacrylate, triethylene glycol dimethacrylate (TEGDMA), ethylene glycol diacrylate, diethyleneglycol diacrylate, buthylene glycol dimethacrylate, buthylene glycol diacrylate, neopentyl glycol dimethacrylate, neopentyl glycol diacrylate, 1,3-butanediol dimethacrylate, 1,3-butanediol diacrylate, 1,4-butanediol dimethacrylate, 1,4-butanediol diacrylate, 1,6-hexanediol dimethacrylate, 1,6-hexanediol diacrylate, di-2-methacryloxyethyl-hexametylene dicarbamate, di-2-methacryloxyethyl-trimethylhexametylene dicarbamate, di-2-methacryloxyethyl-dimethylbenzene dicarbamate, di-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl-4-cyclohexyl carbamate, 2,2-bis(4-(2-hydroxy-3-methacryloxy)phenyl)propane (BisGMA), 2,2'-bis(4-methacryloxyphenyl)propane, 2,2'-bis(4-acryloxyphenyl)propane, 2,2'-bis[4(2-hydroxy-3-acryloxyphenyl)propane, 2,2'-bis(4-methacryloxyethoxyphenyl)propane, 2,2'-bis(4-acryloxyethoxyphenyl)-propane, 2,2'-bis(4-methacryloxypropoxyphenyl)propane, 2,2'-bis(4-acryloxypropoxyphenyl)propane, 2,2'-bis(4-methacryloxydiethoxyphenyl)-propane, 2,2'-bis(4-acryloxydiethoxyphenyl)propane, 2,2'-bis[3(4-phenoxy)-2-hydroxypropane-1-methacrylateJpropane, 2,2'-bis[3(4-phenoxy)-2-hydroxypropane-1-acrylate]propane and mixtures thereof.

The matrix may also be made of crosslinkable monomers or polymers such as ε-caprolactone, polycaprolactone, polylactides, polyhydroxyproline, and other biopolymers as well as polyamides, polyurethane, polyethylene, polypropylene, other polyolefins, polyvinyl chloride, polyester, polyether, polyethyleneglycol, polysaccharide, polyacrylonitrile, poly(methyl methacrylate), phenol-formaldehyde, melamine-formaldehyde, and urea-formaldehyde. The matrix may naturally also consist of a mixture of a monomer(s) and a polymer(s).

Dendrimers having 5 to 35 functional groups such as methacrylate or acrylate groups may also be used. Multifunctionality forms highly cross-linked matrix and decreases the creep of the polymer in the long-term use. Examples of suitable dendrimers are given for example in US 5,834,118 (incorporated herein by reference). Dendrimers may particularly be startburst or hyperbranched methacrylated polyesters.

According to an embodiment of the invention, the matrix is selected from the group consisting of methyl methacrylate, hydroxyethyl methacrylate, urethan dimethacrylate, triethylene glycol dimethacrylate, 2,2-bis(4-(2-hydroxy-3-methacryloxy)phenyl)propane, polymethyl methacrylate, starburst methacrylated polyesters, hyperbranched methacrylated polyesters and mixtures thereof.

A typical polymer in dental applications at the moment of filing this application is polymethyl methacrylate (PMMA), especially PMMA having a molecular weight between 13 000 and 996 000 g/mol. More preferably the molecular weight is between 20 000 and 300 000 g/mol, such a molecular weight allowing an especially easy formation of a dense polymer matrix for the finished composite. It is naturally also possible to use mixtures of PMMA's having different molecular weights.

The curing of the matrix band according to the present invention is performed by a known curing process suitable for the selected matrix. The curing may be induced for example by electromagnetic radiation selected from the group consisting of visible light, ultra-violet light, blue light and laser irradiation. According to another embodiment, said matrix is autopolymerizable and the curing is induced by applying an activator on the matrix band. The humidity of air or the oxygen of air may also function as an activator. It is also possible to use matrixes that are stored in low temperatures (under room temperature or below 0°C) after manufacturing and that autopolymerize once the temperature is increased to room temperature. The preferable curing initiation is obtained by radiation with blue light or by laser with the help of initiators and activators for the polymerization and/or crosslinking.

The matrix band according to the present invention may further comprise particulate filler material, such as inert glass, bioactive glass, metal oxides, ceramics, polymers and mixtures thereof. Metal oxides may for example be used as radio or X-ray opaque materials or as coloring materials. It is for example possible to make the matrix band such that it is not further necessary to coat it with another material to make the final outer surface of the dental restoration.

The thickness and stiffness of the matrix band may be varied according to the intended application of said matrix. The variations are well known to a person skilled in the art and it is possible to affect the thickness and stiffness of the matrix band by varying the fibers, their orientation and the nature and amount of the matrix. Typically, the thickness of the matrix band is between 0,05 and 1,5 mm. The cross-section of a matrix band need not to be constant, it may for example be thinner in its edges. The variations in thickness and stiffness affect the mechanical properties of the matrix band and the resulting dental restoration. These variations and their consequences on thickness and stiffness are obvious to persons skilled in the art, and the proper choice can be ascertained by a few simple tests.

The matrix band can be made for example of a glass fiber weave having a thickness of 0,06 mm that has been pre-impregnated as described in patents US 5,846,640 and US 6,197,410 (herein incorporated in reference). The matrix band is placed to cover the axial surfaces of a tooth. The filling composite that is applied into the cavity comes into contact with the matrix band and after curing of the matrix it will be attached to the filling composite. The matrix band remains on the axial surfaces of the tooth and will usually be covered with a layer of a filling composite to form a polishable and tooth colored surface on the matrix band. The matrix band behaves as a capsule on the tooth and protects the remaining parts of tooth, such as cusps, against fractures.

The matrix band according to the present invention may be applied with a specific tool or with fingers. An example of a tool is presented below in connection with the drawing.

The present invention thus relates to a matrix band in the sense that it is generally understood in the art. The present inventors have however also noticed that the matrix band can also be used in other applications than dental restoration. These applications are more extensively explained below.

The present invention also relates to a dental restoration kit comprising a matrix band according to the present invention, a restorative dental composite and an adhesive. By restorative dental composite it is meant normal tooth filling material. The adhesive is used to attach the matrix band to the tooth to be restored, although the matrix band may be sticky in itself and does not necessarily need a separate adhesive. In general, adhesives are however used. The kit may further comprise an applicator device for positioning said matrix band into place. The matrix band may also be pre-shaped in the form of a dental restoration, a dental crown or a dental bridge, for a more convenient use. It is possible to pre-shape the matrix band to any form and size.

The invention further concerns a prepreg comprising fibers and a matrix, at least a portion of said matrix being at least partially uncured, for use as a dental matrix band. The examples of suitable materials for fibers and matrix given above apply to this embodiment of the invention also. The prepreg according to the present invention may also be used in all the applications mentioned for the matrix band in this specification.

The present invention yet further relates to the use of a matrix band according to the present invention for the manufacturing of a dental restoration, a dental bridge or a dental crown. In these uses, the matrix band forms an integral part of the finished dental restoration, dental bridge or dental crown. The matrix band according to the present invention may also be used as an occlusal matrix band, in order to give support for the occlusal surface of the tooth.

The present invention still relates to the use of a matrix band according to the invention in dental applications. Said application may be selected for example from dental restoration, dental bridge, dental crown, dental restorations and endodontic treatment. The matrix band according to the present invention may advantageously be used in the manufacturing of a micro-invasive crown, since it allows the manufacturing of a crown without substantial drilling of the adjacent teeth.

The matrix band according to the present invention may further be used as encapsulation material of parts of fiber-reinforced composite bridges, as temporary isolation device during endodontic treatment, as a framework for veneering composite resins of dental bridges and crowns or as a repair material for dental restorations.

Some of the above-mentioned uses are discussed more in detail in connection with the drawing below. By temporary isolation device during endodontic treatment, it is meant to use the matrix band according to the present invention to prevent saliva for entering the root canal. Indeed, saliva should not enter said root canal, which is difficult to prevent in cases where the tooth is broken down to the level of the gingiva. The matrix band is temporarily positioned around the end of the root, in order to elevate its upper end to prevent the entering of saliva into the root canal, during the treatment. The matrix band according to the present invention may further be used in any other dental restorations where it is desirable to increase the strength of the finished restoration.

The present invention further relates to a method of manufacturing a dental restoration. The method comprises the following steps:
- drilling a cavity on the tooth to be repaired,
- etching or priming the surface of said tooth,
- applying a matrix band according to the present invention around said tooth, and
- filling said cavity with restorative material.

The method optionally also comprises one or more of the further steps of:
- curing the restorative material,
- covering the thus obtained restoration with a covering material,
- polishing the thus obtained finished restoration.

The matrix band may be cured immediately after it has been applied around the tooth, partly or fully, or it may be cured together with the restorative material, or the curing of the matrix band may be finished at the same time as the restorative material is cured.

The present invention further relates to a method of manufacturing a dental bridge, comprising the steps of:
- preparation of teeth
- etching or priming the surface of the abutment teeth adjacent to the crown to be manufactured,
- positioning a fiber framework on said abutment teeth,
- positioning a matrix band according to the present invention on each of said abutment teeth,
- manufacturing of a pontic,
- finishing the esthetic look of the pontic and the adjacent teeth, and
- polishing the dental bridge obtained.

The method may additionally comprise the step of covering the matrix band with a layer of filling composite resin or other suitable material. The matrix band and/or the fiber framework may be cured immediately after it/they has been applied on the teeth, partly or fully, or it/they may be cured together with the pontic. The manufacturing of a pontic is made according to any method known in the art. The step of finishing the esthetic look of the pontic and the adjacent teeth typically includes layering and/or covering the pontic and optionally of the adjacent teeth, for example with a composite layering technique.

The present invention further relates to a method of manufacturing a micro-invasive dental crown, comprising the steps of:
- preparation of the tooth,
- etching or priming the remaining surface of said tooth,
- applying a matrix band according to the present invention around said tooth,
- manufacturing an artificial crown within said matrix band,
- curing the material of said crown,
- applying a layer of surface material on said cured crown, and
- polishing the surface of the obtained finished crow.

The matrix band may be cured immediately after it has been applied around the tooth, partly or fully, or it may be cured together with the manufacturing of the artificial crown.

In this specification, except where the context requires otherwise, the words "comprise", "comprises" and "comprising" means "include", "includes" and "including", respectively. That is, when the invention is described or defined as comprising specified features, various embodiments of the same invention may also include additional features. Also, the reference signs should not be construed as limiting.

The invention is described below in greater detail by the following, non-limiting drawings.

### BRIEF DESCRIPTION OF THE DRAWING

Figure 1 illustrates schematically the use of a matrix band according to a first embodiment of the invention.
Figure 2 illustrates schematically the use of a matrix band according to a second embodiment of the invention.
Figure 3 illustrates schematically the use of a matrix band according to a third embodiment of the invention.
Figure 4 illustrates schematically the use of a matrix band according to a fourth embodiment of the invention.
Figure 5 illustrates schematically a device for placing a matrix band according to the invention.
Figure 6 illustrates schematically the use of a matrix band according to a fifth embodiment of the invention.
Figure 7 illustrates the enclosed Example.

### DETAILLED DESCRIPTION OF THE DRAWING

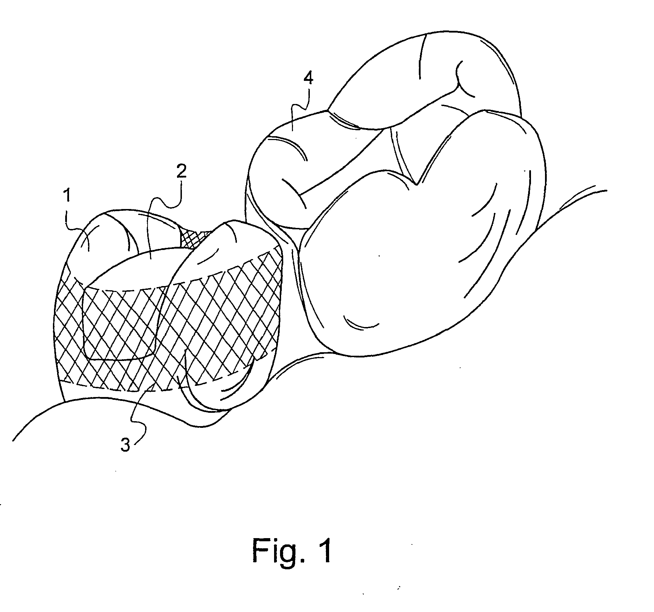

Figure 1 illustrates schematically the use of a matrix band according to a first embodiment of the invention. The drawing illustrates a premolar tooth 1 having mesiodistal cavity 2 to be filled with the restorative composite resin (not shown). A fiber-reinforced composite matrix band 3 is placed around the tooth 1 offering support for the restorative composite resin to be applied to the cavity 2. When the restorative composite resin is cured, the composite resin is bonded to the matrix band 3 that will remain as a part of tooth-filling system. Approximal contact to the tooth 4 behind the restored premolar tooth 1 remains without gap formation because the matrix band 3 is not removed from the tooth 1 after curing the composite resin.

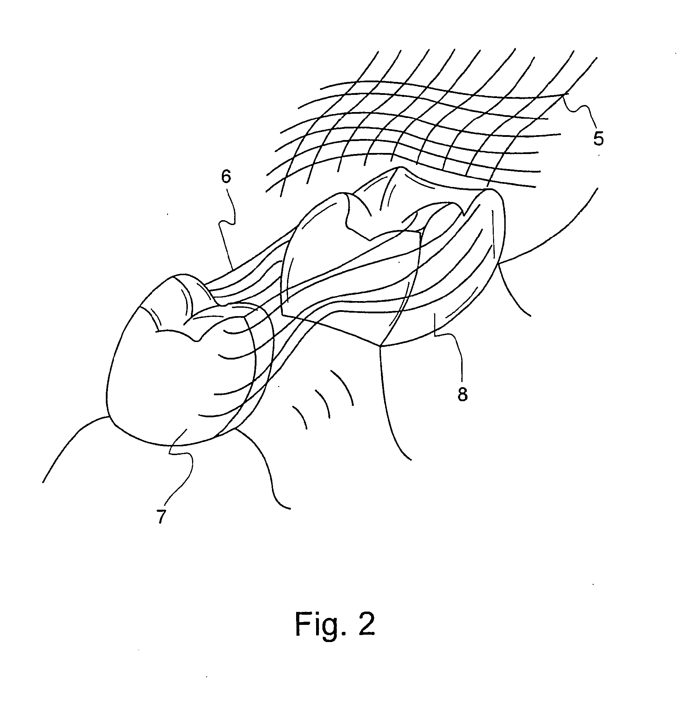

Figure 2 illustrates schematically the use of a matrix band according to a second embodiment of the invention. In this embodiment, a matrix band is used to reinforce a fiber composite bridge. The matrix band 5 is placed over of a tooth to encapsulate and protect the fibers of the framework 6.

To protect the fibers of a continuous unidirectional fiber composite framework 6 of a bridge on abutment teeth 7 and 8, a matrix band 5 is placed on the fibers of the framework 6. The matrix band 5 is here shown above the framework 6 for sake of clarity. The matrix band 5 forms, after curing, a capsule on the framework fiber - tooth system. In addition to the protection effect of the matrix band 5, the matrix band 5 also transfers stresses to larger surface area of the abutment teeth 7, 8 and thus reduces the debonding stresses. The matrix band 5 is preferably covered with a layer of filling composite resin where there is room and need for the composite material in the occlusion.

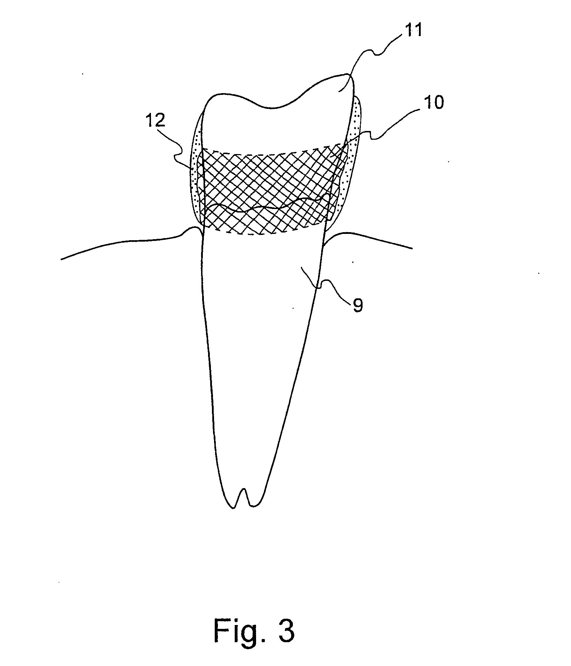

Figure 3 illustrates schematically the use of a matrix band according to a third embodiment of the invention. More precisely, the Figure illustrates a fractured tooth repaired with a crown supported by a matrix band. Firstly, a matrix band 10 is placed on the root of a premolar 9 and cured. An artificial crown 11 is added inside the matrix band 10 and formed to the anatomical shape of a crown. After the material of the crown 11 has been cured, it is attached to the matrix band 10 that supports the crown-root-system. A layer of a restorative composite resin 12 is then placed over the matrix band 10 to offer a polishable surface for the finished crown.

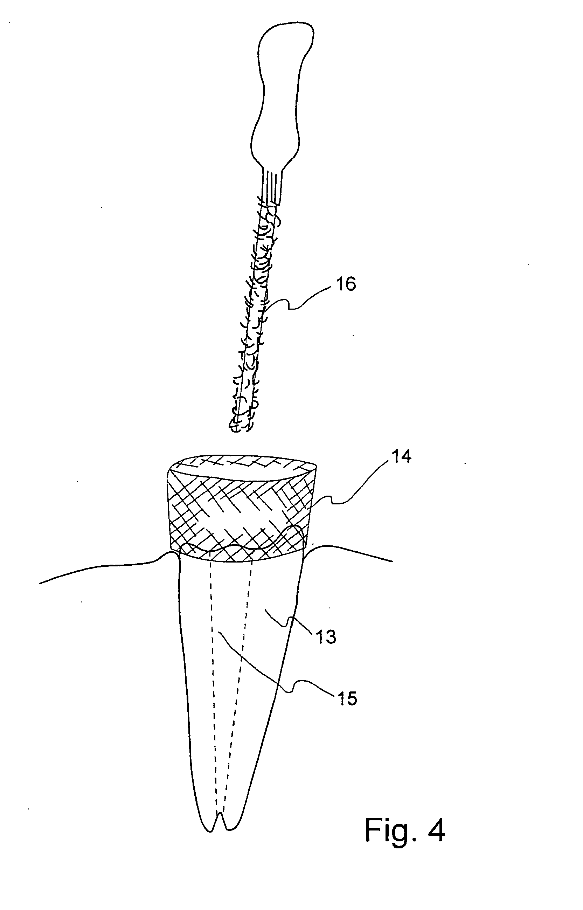

Figure 4 illustrates schematically the use of a matrix band according to a fourth embodiment of the invention, in endodontic treatment to protect the root canal from saliva contamination during the treatment. A premolar 13 needing endodontic treatment is firstly restored by a matrix band according to the present invention, thus forming a funnel 14 that prevents the saliva from entering the root canal 15. The thus formed funnel 14 allows aseptic endodontic treatment to be performed in a manner known *per se,* with endodontic instruments 16.

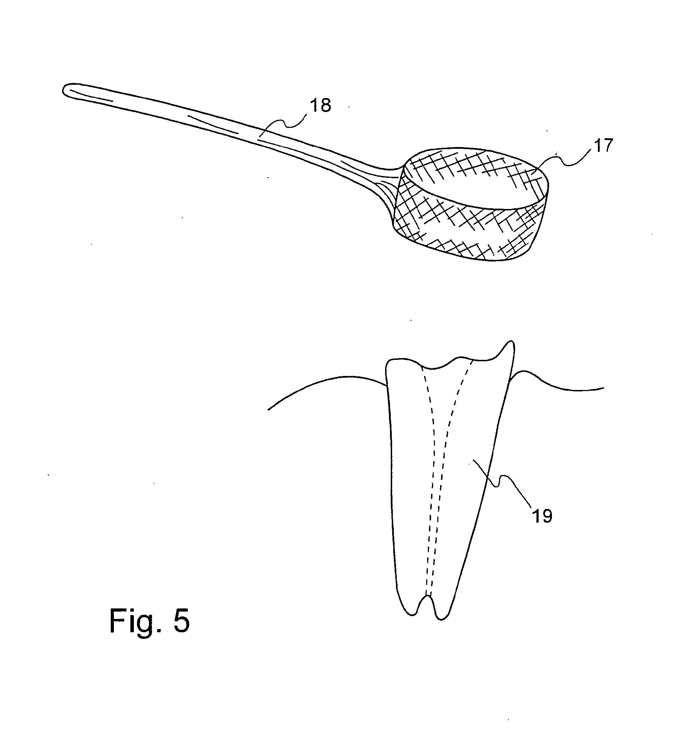

Figure 5 illustrates schematically a device for the positioning of a matrix band according to the invention. The matrix band 17 is arranged to a device 18 that allows the easy positioning of said matrix band 17 on the tooth 19.

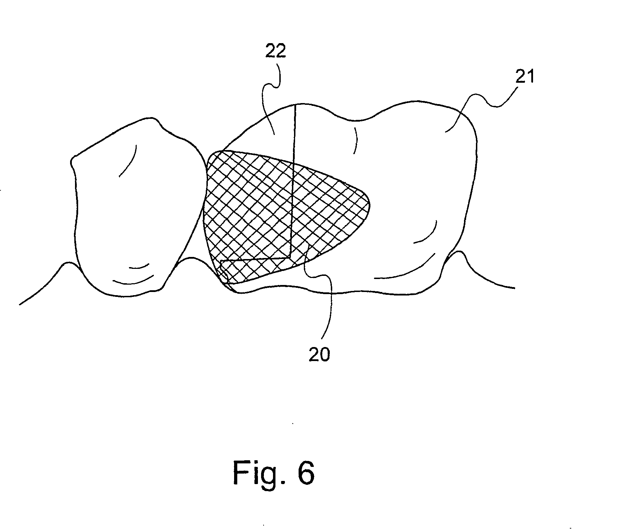

Figure 6 illustrates schematically the use of a matrix band according to a fifth embodiment of the invention. More specifically, the Figure illustrates a matrix band 20 according to the present invention in the manufacturing of a mesial filling of a molar tooth 21. In this embodiment, the matrix band 20 is used as a partial covering in the restoration of a decayed molar tooth 21 needing a mesial filling 22. The matrix band 20 is placed on the tooth and cured. When the filling material is cured, it attaches to the matrix band 20 thus forming a filling-matrix band-system that supports the tooth against new fractures.

### EXPERIMENTAL PART

The matrix band according to the present invention was tested in a dental restoration. The resulting restoration was tested against cusp fracture and compared to the cusp fracture of a restoration made in a conventional manner.

A buccal cusp of a premolar tooth was drilled away and restorated with Z250 restorative composite resin (3M Espe). One restoration was manufactured without a matrix band according to the present invention (A) and one restoration was manufactured with a matrix band according to the present invention (B).

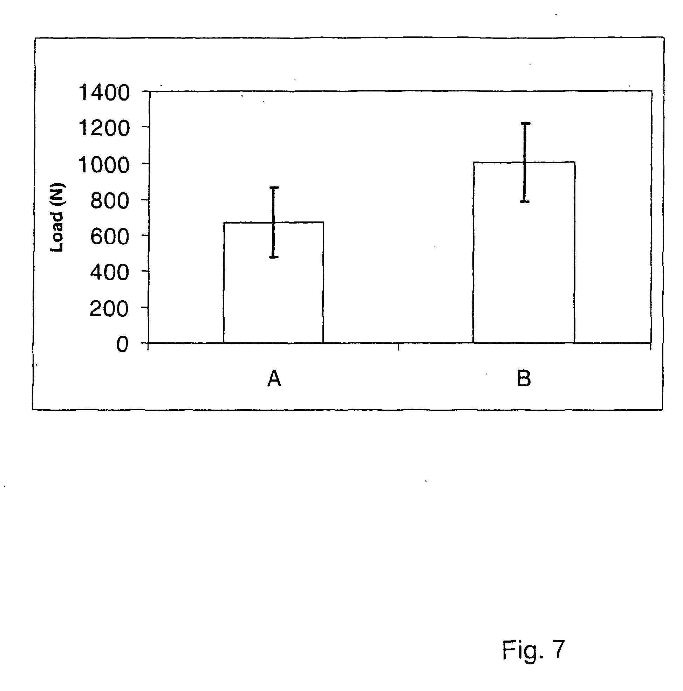

The repaired cusps were then loaded in 45° with a material testing machine (Model LRX, Lloyd Instruments) and the results are shown in Figure 7. It can be seen that the tooth having a matrix band according to the present invention (B) was approximately 30 % stronger that the tooth without said matrix band (A).

## Claims

1. A matrix band, **characterized in that** it consists essentially of fibers and a matrix, at least a portion of said matrix being at least partially uncured.

2. Matrix band according to claim 1, **characterized in that** said matrix is selected from the group consisting of methyl methacrylate, hydroxyethyl methacrylate, urethan dimethacrylate, triethylene glycol dimethacrylate, 2,2-bis(4-(2-hydroxy-3-methacryloxy)phenyl)propane, polymethyl methacrylate, starburst methacrylated polyesters, hyperbranched methacrylated polyesters and mixtures thereof.

3. Matrix band according to any of the preceding claims, **characterized in that** said fibers are selected from the group consisting of inert glass fibers, bioactive glass fibers, silica fibers, quartz fibers, ceramic fibers, carbon/graphite fibers, aramid fibers, ceramic fibers, poly(p-phenylene-2,6-benzobisoxazole) fibers, poly(2,6-diimidazo(4,5-b4',5'-e)pyridinylene-1,4(2,5-dihydro)phenylene fibers, polyolefin fibers, fibers prepared from copolymers of olefins, polyester fibers, polyamide fibers, polyacrylic fibers, sol-gel processed silica fibers, collagen fibers, cellulose fibers, modified cellulose fibers and mixtures thereof.

4. Matrix band according to any of the preceding claims, **characterized in that** said fibers are in the form of continuous fibers, chopped fibers, mat, sheet or mixtures thereof, and **in that** they are oriented in one, two, three or four directions, randomly or mixtures thereof.

5. Matrix band according to any of the preceding claims, **characterized in that** it further comprises particulate filler material.

6. Matrix band according to claim 5, **characterized in that** said particulate filler material is selected from the group consisting of inert glass, bioactive glass, metal oxides, ceramics, polymers and mixtures thereof.

7. A dental restoration kit comprising a matrix band according to any of the claims 1-6, a restorative dental composite and an adhesive.

8. Kit according to claim 7, **characterized in that** it further comprises an applicator device.

9. Kit according to claim 7 or 8, **characterized in that** said matrix band is pre-shaped in the form of a dental restoration, a dental crown or a dental bridge.

10. Use of a matrix band according to any of the claims 1-6 for the manufacturing of a dental restoration, a dental bridge or a dental crown.

11. Use according to claim 10, **characterized in that** said matrix band forms an integral part of the finished dental restoration, dental bridge or dental crown.

12. Use of a matrix band according to any of the claims 1-6 in dental applications.

13. Use according to claim 12, **characterized in that** said application is selected from dental restoration, dental bridge, dental crown and endodontic treatment.

## Patentansprüche

1. Matrixstreifen, **dadurch gekennzeichnet, dass** er im Wesentlichen aus Fasern und einer Matrix besteht, wobei mindestens ein Teil der Matrix zumindest teilweise ungehärtet ist.

2. Matrixstreifen gemäß Anspruch 1, **dadurch gekennzeichnet, dass** die Matrix ausgewählt ist aus der Gruppe, bestehend aus Methylmethacrylat, Hydroxyethylmethacrylat, Urethandimethacrylat, Triethylenglykoldimethacrylat, 2,2-Bis(4-(2-hydroxy-3-methacryloxy)phenyl)propan, Polymethylmethacrylat, strahlenförmig bzw. starburst methacrylierten Polyestern, hyperverzweigten methacrylierten Polyestern und Gemischen davon.

3. Matrixstreifen gemäß einem der vorhergehenden Ansprüche, **dadurch gekennzeichnet, dass** die Fasern ausgewählt sind aus der Gruppe, bestehend aus inerten Glasfasern, bioaktiven Glasfasern, Silicafasern, Quarzfasern, Keramikfasern, Kohlenstoff/Graphit-Fasern, Aramidfasern, Keramikfasern, Poly(p-phenylen-2,6-benzobisoxazol)-Fasern, Poly(2,6-diimidazo(4,5-b4',5'-e)pyridinylen-1,4(2,5-dihydro)phenylen-Fasern, Polyolefinfasern, Fasern, hergestellt aus Copolymeren von Olefinen, Polyesterfasern, Polyamidfasern, Polyacrylfasern, Sol-Gel-verarbeiteten Silicafasern, Kollagenfasern, Cellulosefasern, modifizierte(n) Cellulose-Fasern und Gemischen davon.

4. Matrixstreifen gemäß einem der vorhergehenden Ansprüche, **dadurch gekennzeichnet, dass** die Fasern in Form von kontinuierlichen Fasern, Kurzfasern, einer Matte bzw. einem Vlies, einem Blatt bzw. einer Folie oder Mischungen davon vorliegen und dass sie in ein, zwei, drei oder vier Richtungen, zufällig oder als Mischungen davon orientiert sind.

5. Matrixstreifen gemäß einem der vorhergehenden Ansprüche, **dadurch gekennzeichnet , dass** er weiterhin partikuläres Füllstoffmaterial umfasst.

6. Matrixstreifen gemäß Anspruch 5, **dadurch gekennzeichnet, dass** das partikuläre Füllstoffmaterial ausgewählt ist aus der Gruppe, bestehend aus inertem Glas, bioaktivem Glas, Metalloxiden, Keramiken, Polymeren und Gemischen davon.

7. Dentalrestorationskit, umfassend einen Matrixstreifen gemäß einem der Ansprüche 1 bis 6, einen Dentalrestorationsverbundstoff und ein Klebemittel.

8. Kit gemäß Anspruch 7, **dadurch gekennzeichnet, dass** er weiterhin eine Applikatorvorrichtung umfasst.

9. Kit gemäß Anspruch 7 oder 8, **dadurch gekennzeichnet, dass** der Matrixstreifen in der Form einer Dentalrestoration, einer Zahnkrone oder einer Zahnbrücke vorgeformt ist.

10. Verwendung eines Matrixstreifens gemäß einem der Ansprüche 1 bis 6 zur Herstellung einer Dentalrestoration, einer Zahnbrücke oder einer Zahnkrone.

11. Verwendung gemäß Anspruch 10, **dadurch gekennzeichnet, dass** der Matrixstreifen einen integralen Teil der fertigen Dentalrestoration, Zahnbrücke oder Zahnkrone bildet.

12. Verwendung eines Matrixstreifens gemäß einem der Ansprüche 1 bis 6 in zahnärztlichen Applikationen.

13. Verwendung gemäß Anspruch 12, **dadurch gekenn- zeichnet**, dass die Applikation ausgewählt ist aus Dentalrestoration, Zahnbrücke, Zahnkrone und endodontischer Behandlung.

## Revendications

1. Bande de matrice, **caractérisée en ce qu'**elle est constituée essentiellement par des fibres et une matrice, au moins une partie de ladite matrice étant au moins partiellement non durcie.

2. Bande de matrice selon la revendication 1, **caractérisée en ce que** ladite matrice est choisie dans le groupe constitué par le méthacrylate de méthyle, le méthacrylate d'hydroxyéthyle, le diméthacrylate d'uréthanne, le diméthacrylate de triéthylèneglycol, le 2,2-bis(4-(2-hydroxy-3-méthacryloxy)phényl)propane, le poly(méthacrylate de méthyle), des polyesters méthacrylés de type étoilé, des polyesters méthacrylés hyperramifiés et leurs mélanges.

3. Bande de matrice selon l'une quelconque des revendications précédentes, **caractérisée en ce que** lesdites fibres sont choisies dans le groupe constitué par les fibres de verre inerte, les fibres de verre bioactif, les fibres de silice, les fibres de quartz, les fibres de céramique, les fibres de carbone/graphite, les fibres aramides, les fibres de céramique, les fibres de poly(p-phénylène-2,6-benzobisoxazole), les fibres de poly(2,6-diimidazo(4,5-b4',5'-e)pyridinylène-1,4(2,5-dihydro)phénylène, les fibres de polyoléfine, les fibres préparées à partir de copolymères d'oléfines, les fibres de polyester, les fibres de polyamide, les fibres polyacryliques, les fibres de silice traitées par procédé sol-gel, les fibres de collagène, les fibres cellulosiques, les fibres cellulosiques modifiées et leurs mélanges.

4. Bande de matrice selon l'une quelconque des revendications précédentes, **caractérisée en ce que** lesdites fibres sont sous la forme de fibres continues, de fibres coupées, de mats, de feuilles ou leurs mélanges, et **en ce qu'**elles sont orientées vers une, deux, trois ou quatre directions, de manière aléatoire ou leurs mélanges.

5. Bande de matrice selon l'une quelconque des revendications précédentes, **caractérisée en ce qu'**elle comprend en outre une matière de charge particulaire.

6. Bande de matrice selon la revendication 5, **caractérisée en ce que** ladite matière de charge particulaire est choisie dans le groupe constitué par le verre inerte, le verre bioactif, les oxydes métalliques, les céramiques, les polymères et leurs mélanges.

7. Trousse de restauration dentaire comprenant une bande de matrice selon l'une quelconque des revendications 1 à 6, un composite de restauration dentaire et un adhésif.

8. Trousse selon la revendication 7, **caractérisée en ce qu'**elle comprend en outre un dispositif applicateur.

9. Trousse selon la revendication 7 ou 8, **caractérisée en ce que** ladite bande de matrice est préformée sous la forme d'une restauration dentaire, d'une couronne dentaire ou d'un bridge dentaire.

10. Utilisation d'une bande de matrice selon l'une quelconque des revendications 1 à 6, pour la fabrication d'une restauration dentaire, d'un bridge dentaire ou d'une couronne dentaire.

11. Utilisation selon la revendication 10, **caractérisée en ce que** ladite bande de matrice fait partie intégrante de la restauration dentaire, du bridge dentaire ou de la couronne dentaire achevés.

12. Utilisation d'une bande de matrice selon l'une quelconque des revendications 1 à 6, dans des applications dentaires.

13. Utilisation selon la revendication 12, **caractérisée en ce que** ladite application est choisie parmi la restauration dentaire, le bridge dentaire, la couronne dentaire et le traitement endodontique.
